# EUROPÄISCHE PATENTSCHRIFT

(11) **EP 1 770 179 B1**
(45) Veröffentlichungstag und Bekanntmachung des Hinweises auf die Patenterteilung: **24.09.2008**
(21) Anmeldenummer: 05107053.0
(22) Anmeldetag: 29.07.2005
(51) Int. Cl.: C22C 5/06, A61K 6/04

(54) **Aufbrennfähige Silber-Legierung zur Herstellung keramisch verblendeter Dentalrestaurationen**
Fireable silver alloys for the manufacture of ceramic lined dental restorations
Alliage d'argent à cuire pour la fabrication de restauration dentaire revetue de ceramique.

(43) Veröffentlichungstag der Anmeldung: 04.04.2007
(73) Patentinhaber: BEGO Bremer Goldschlägerei Wilh. Herbst GmbH & Co. KG, 28359 Bremen (DE)
(72) Erfinder: Strietzel, Roland, 28865, Lilienthal (DE)
(74) Vertreter: Eisenführ, Speiser & Partner

(56) Entgegenhaltungen:
- EP-A2- 0 475 528
- DE-A1- 2 908 203
- DE-U1- 20 220 836
- US-A1- 2002 122 741

## Beschreibung

Die Erfindung betrifft eine aufbrennfähige Legierung zur Herstellung keramisch verblendbarer Dentalrestaurationen, beispielsweise für die Herstellung von Kronen, Brücken, Suprakonstruktionen und anderen Zahnprothesen, die mit einer Keramikoberfläche versehen werden sollen, aber auch für unverblendete Restaurationen wie Inlays, Onlays, Hilfsteile etc.

Die Kostenentwicklung im Gesundheitswesen führt zu einem hohen Druck auch auf die Materialkosten. Im Bereich der Zahnheilkunde werden neben edelmetallfreien auch Edelmetall (EM-) Legierungen verwendet. Ihr Einsatzgebiet umfasst insbesondere Inlays, Onlays sowie Kronen- und Brückengerüste. Die letztgenannten Gerüste können völlig oder teilweise mit Keramik oder Kunststoff verblendet werden. Durch den hohen Preis der Rohstoffe von EM-Legierungen (z. B. Gold, Platin) ist diese Legierungsgruppe für beträchtliche Teile der Bevölkerung inakzeptabel teuer. Es wird daher beständig nach kostengünstigen EM-Legierungen gesucht. In diesem Zusammenhang ist der Einsatz von Silber-Legierungen eine vielfach geeignete Alternative.

Silberhaltige EM-Legierungen sind insbesondere aus den folgenden Dokumenten bekannt:

WO 03/028669 A1, EP 1 346 718 A1, EP 1 340 482 A1, EP 0 973 480 B1, NL 9200566, US 6,290,501 B1, US 3,929,474, US 4,008,080, DE 100 33 445 A1, US 5,453,290, EP 475 528 B2 und DE 2 908 203.

Auch im Handel sind eine Reihe von aufbrennfähigen Legierungen auf Silber-Basis erhältlich. In den meisten Fällen handelt es sich um Silber-PalladiumLegierungen, d. h. der Legierungsbestandteil mit dem zweitgrößten Anteil ist meist Palladium. In manchen Fällen ist Gold der Legierungsbestandteil mit dem zweitgrößten Anteil.

Aufbrennfähige Silber-Legierungen werden in der Praxis häufig mit Keramiken verblendet, deren linearer Wärmeausdehnungskoeffizient (WAK-Wert) im Bereich von etwa 15-18 [10⁻⁶ K ⁻¹] im Bereich von 25 bis 500 °C liegt. Auch die erfindungsgemäße aufbrennfähige Legierung ist zur Verblendung mit derartigen Verblendkeramiken vorgesehen.

Bei der Konzeption neuer aufbrennfähiger Legierungen auf Silber-Gold-Basis muss der Fachmann eine Vielzahl technischer Eigenschaften berücksichtigen und versuchen, ausgewählte Eigenschaften besonders günstig einzustellen, ohne dabei die anderen Eigenschaften in besonders nachteiliger Weise zu beeinflussen.

Neben den mechanischen Eigenschaften müssen auch die chemischen und biologischen beachtet werden. Zu den mechanischen Eigenschaften gehören:
- Mechanische Festigkeit (Elastizitätsmodul, 0,2 % Dehngrenze, Zugfestigkeit, Bruchdehnung)
- Warmfestigkeit
- Wärmeausdehnungskoeffizient (WAK)
- Härte

Zu den zu beachtenden chemischen Eigenschaften gehören die Korrosion und die Verfärbungen. Besonders letztere können sehr problematisch werden. Silber neigt zur Reaktion z. B. mit Sulfiden und anderen Schwefel-haltigen Gruppen. Das Reaktionsprodukt, Silbersulfid, ist schwarz gefärbt und stört nachhaltig den ästhetischen Eindruck.

Die biologischen Eigenschaften (toxische oder allergische Reaktionen) hängen vorwiegend vom Korrosionsverhalten ab. Daher muß die lonenabgabe so gering wie möglich gehalten werden. Aber auch die Art der abgegebenen Ionen ist zu beachten. So wirken Kupfer- und Silber-Ionen bakterizid, was grundsätzlich positiv zu bewerten ist. Allerdings wirken diese Elemente bei zunehmender Konzentration zelltoxisch, wodurch lokal-toxische Reaktionen verursacht werden können.

Angesichts der vorstehend beschriebenen Probleme bei der Verwendung herkömmlicher Silber-Legierungen war es die der vorliegenden Erfindung zu Grunde liegende Aufgabe, eine Silber-Legierung anzugeben, (i) bei der der Gießzeitpunkt eindeutig erkennbar ist, (ii) die nach dem Abguss eine besonders gute Passung aufweist (hohe Passgenauigkeit der aus der Legierung gegossenen Gerüste (Dentalrestaurationen), insbesondere bei großer Spannweite), (iii) die mit den üblichen Verblendkeramiken mit einem WAK im Bereich 15-17 [10⁻⁶ K⁻¹] verblendbar sind, (iv) eine hohe Korrosionsresistenz besitzen und (v) biokompatibel sind. Weitere der vorliegenden Erfindung zu Grunde liegende Aufgabenstellungen ergeben sich aus der nachfolgenden Beschreibung und den beigefügten Patentansprüchen.

Für die anzugebende Silber-Legierung sollten die sonstigen für eine aufbrennfähige Dentallegierung wichtigen Legierungsmerkmale bzw. Legierungseigenschaften innerhalb der vom Fachmann bevorzugten Bereiche liegen.

Erfindungsgemäß wird die der Erfindung zu Grunde liegende Aufgabe gelöst durch eine aufbrennfähige Legierung zur Herstellung keramisch verblendeter Dentalrestaurationen, bestehend aus:

| | |
|---|---|
| Silber | 40-45 Gew.-%, |
| Gold | 37,5-45 Gew.-%, |
| Palladium | 0-14,5 Gew.-%, |
| Indium, Zink, Zinn, Kupfer, Lanthanoide, Scandium, Lanthan | insgesamt 5,5-15 Gew.-%, |
| Mangan | 0,1-5 Gew.-%, |
| Tantal, Platin, Iridium, Osmium, Ruthenium, Rhodium, Rhenium, Titan, Niob, Zirkonium, Wolfram, Vanadium | insgesamt 0,05-5 Gew.-%, |
| Gallium, Eisen | 0-5 Gew.-%, |
| sonstige Metalle, Halbmetalle und Verunreinigungen | 0-1 Gew.-%, |

wobei die Gewichtsprozentangaben jeweils bezogen sind auf das Gesamtgewicht der Legierung.

Die erfindungsgemäße aufbrennfähige Legierung enthält einen Anteil von 40-45 Gew.-% Silber. Aufgrund des hohen Silberanteils ist ein für eine EM-Legierung vergleichsweise günstiger Materialpreis erreichbar. Silber legt als Hauptbestandteil der erfindungsgemäßen Legierung deren grundlegende mechanische und chemische Eigenschaften fest. Aufgrund des hohen Gehaltes an Silber kann der WAK der erfindungsgemäßen aufbrennfähigen Legierung an die WAK von hochexpandierenden Keramiken, sogenannten LFC-Massen (low fusing ceramic) angepasst werden. Vorzugsweise beträgt der Anteil an Silber in einer erfindungsgemäßen Legierung 40-44 Gew.-%, bevorzugt 40,3-43,0 Gew.-%.

In einer erfindungsgemäßen Legierung beträgt der Anteil an Gold 38-42 Gew.-%, vorzugsweise jedoch 38-40 Gew.-%. Aufgrund der guten Mischbarkeit von Gold und Silber kommt es in einer erfindungsgemäßen Legierung zu einer homogenen Kristallstruktur und zu einer Mischkristallverfestigung. Aufgrund des noch edleren Charakters des Goldes im Vergleich mit Silber ist die Korrosionsresistenz einer erfindungsgemäßen Legierung besonders hoch. Der Anteil an Gold führt zudem zu einem optimierten Farbeindruck; eine polierte Oberfläche einer erfindungsgemäßen Legierung zeigt einen gefälligen warmen Farbton.

Der Anteil an Palladium in einer erfindungsgemäßen aufbrennfähigen Legierung liegt im Bereich von 0-14,5 Gew.-%. Vorzugsweise liegt der Anteil an Palladium in einer erfindungsgemäßen Legierung im Bereich von 12,5-14,5 Gew.-%. Eine Zugabe von Palladium erhöht (i) die mechanische Festigkeit der Legierung und (ii) die Lage des Schmelzintervalls. Ein Palladiumgehalt über 14,5 Gew.-% würde allerdings in inakzeptabler Weise entfärbend wirken und einen gelblich-warmen Farbton unmöglich machen.
Die Einstellung der bevorzugten Konzentrationsbereiche für Silber, Gold und Palladium führt unabhängig voneinander zu besonders vorteilhaften erfindungsgemäßen Legierungen. Besonders bevorzugt sind jedoch erfindungsgemäße Legierungen, in denen jedes der Elemente Silber, Gold und Palladium eine Konzentration in den zuvor als bevorzugt angegebenen Bereichen besitzt.

Der Gesamtanteil an Indium, Zink, Zinn, Kupfer, Lanthanoiden, Scandium und Lanthan in einer erfindungsgemäßen Legierung liegt im Bereich von 5,5-15 Gew.-%. In diesem Mengenbereich tragen diese Elemente dazu bei, die mechanische Festigkeit der Silber-Gold-Palladium-Basis einer erfindungsgemäßen Legierung auf die erforderlichen Werte zu erhöhen. Überdies lässt sich mit einer erfindungsgemäßen Legierung aufgrund des erfindungsgemäß vorhandenen Anteils an den genannten Elementen ein günstiger Haftverbund zu üblichen Verblendkeramiken herstellen. Weiterhin erhöhen die genannten Elemente in gewünschter Weise die Härte der Legierung.

Ein Anteil an den genannten Elementen von mehr als 15 Gew.-% würde hingegen zu einer inakzeptabel hohen Sprödigkeit des fertigen Produktes (Gussstückes) und beim zahntechnischen Gerüst zur Bildung von Oxidschichten auf der Schmelze führen, welche die Bestimmung des Gießzeitpunktes erschweren würden. Bei viel höheren als den erfindungsgemäß vorhandenen Anteilen an den genannten Elementen würden sich zudem mehrphasige Gefüge ausbilden, insbesondere palladiumhaltige Phasen. In der Mundhöhle neigen solche Phasen im Laufe der Zeit zu dunklen Verfärbungen; Daher muss ihre Bildung unterdrückt werden. Auch würde bei einem zu hohen Anteil an den genannten Elementen der Wärmeausdehnungskoeffizient einer entsprechenden Legierung in vielen Fällen ungünstig beeinflusst werden, was eine Inkompatibilität mit üblichen Dentalkeramiken nach sich ziehen würde.

Der Einsatz von weniger als 5,5 Gew.-% an den genannten Elementen Indium, Zink, Zinn, Kupfer, Lanthanoiden, Scandium bzw. Lanthan würde nicht mehr zu den oben genannten, gewünschten Effekten (Beeinflussung von Härte, Festigkeit und Haftverbund) führen.

Vorzugsweise liegt der Anteil an den genannten Elementen in einer erfindungsgemäßen Legierung im Bereich von 5,5-10 Gew.-%. Schon bei Anteilen über 10 Gew.-% beginnen die negativen Effekte hoher Gehalte dieser Elemente spürbar zu werden.

Bevorzugt ist der Einsatz von Indium; vorzugsweise umfasst eine erfindungsgemäße Legierung deshalb 5-15 Gew.%, vorzugweise 5,5-10 Gew.-% Indium. Vorzugsweise umfasst eine erfindungsgemäße Legierung dann kein Zink, Zinn, Kupfer, Lanthanoid, Scandium oder Lanthan.

Eine erfindungsgemäße aufbrennfähige Legierung umfasst einen Anteil an Mangan im Bereich von 0,1-5 Gew.-%. Mangan fungiert in einer erfindungsgemäßen Legierung als Sauerstofffänger, Haftoxidbildner und Entschwefelungsmittel. Es bewirkt zudem eine günstige Oxidfarbe. Überdies trägt Mangan dazu bei, dass die Viskosität einer Schmelze einer erfindungsgemäßen Legierung recht niedrig ist. Außerdem wird durch die Anwesenheit des Mangans eine hohe Festigkeit und eine gute Polierbarkeit der Legierung erreicht. Ein Schmieren einer erfindungsgemäßen Legierung während des Politur-Vorganges, wie es bei einigen hochgoldhaltigen Legierungen zu beobachten ist, tritt aufgrund der Anwesenheit des Mangans nicht auf.

Ein Anteil an Mangan von mehr als 5 Gew.-% würde zu einer unerwünschten Erhöhung des WAK-Wertes einer entsprechenden (nicht erfindungsgemäßen) Legierung und zu einem ungünstigeren Gießverhalten führen. Ein zu hoher Mangangehalt könnte zudem zu unerwünschten Reaktion mit der beim Gießen verwendeten Einbettmasse oder der aufzubrennenden Keramik führen. Es könnten sich eutektische Verbindungen mit Silizium bilden, die durch die Einlagerung von Mangandioxid (= Braunstein) braun gefärbt sein und in die Tiefe der Keramik diffundieren können und somit den ästhetischen Eindruck zerstören würden.

Ein Anteil an Mangan von weniger als 0,1 Gew.-% würde dazu führen, dass die vorstehend für eine erfindungsgemäße Legierung genannten Funktionen des Mangans im Einzelfall nicht mehr zur vollen Zufriedenheit erreicht würden.

Vorzugsweise beträgt der Anteil an Mangan in einer erfindungsgemäßen Legierung 0,1-1 Gew.-%.

In einer erfindungsgemäßen Legierung liegt der Gesamtanteil an Tantal, Platin, Iridium, Osmium, Rhutenium, Rhodium, Rhenium, Titan, Niob, Zirkonium, Wolfram und Vanadium im Bereich von 0,05-5 Gew.-%. Die genannten Elemente wirken als Kornfeiner und tragen zur hohen mechanischen Festigkeit der erfindungsgemäßen Legierung bei. In Konzentrationen über 5 Gew.-% würden genannten Metallen in einer erfindungsgemäßen Legierung sogar nicht höher als 0,2 Gew.-%, bereits in derart niedrigen Konzentrationen treten die gewünschten Effekte auf.

Der Einsatz von Tantal ist besonders bevorzugt; deshalb umfasst eine erfindungsgemäße Legierung vorzugsweise 0,05-5 Gew.-% Tantal, bevorzugt sogar nur 0,05-0,2 Gew.-%.

Vorzugsweise umfasst eine erfindungsgemäße Legierung insgesamt nicht mehr als 1 Gew.-% an Zink, Zinn, Kupfer, Lanthanoiden, Scandium, Lanthan, Platin, Iridium, Osmium, Rhutenium, Rhodium, Rhenium, Titan, Niob, Zirkonium, Wolfram und Vanadium.

Ein ganz bevorzugtes Ausführungsbeispiel für eine erfindungsgemäße Legierung bildet die folgende Zusammensetzung:

| | |
|---|---|
| Silber | 40,5 Gew.-% |
| Gold | 38,1 Gew.-% |
| Palladium | 13,0 Gew.-% |
| Indium | 8,0 Gew.-% |
| Palladium | 13,0 Gew.-% |
| Indium | 8,0 Gew.-% |
| Mangan | 0,3 Gew.-% |
| Tantal | 0,1 Gew.-% |

Für die ganz bevorzugte erfindungsgemäße Legierung wurden folgende Legierungseigenschaften bestimmt:

| | |
|---|---|
| Dichte [g/cm³] | ca. 11 |
| Vickershärte [HV 5] | 210 |
| Elastizitätsmodul [GPa] | 120 |
| Dehngrenze (Rp 0,2) [MPa] | 491 |
| Zugfestigkeit [MPa] | 663 |
| Bruchdehnung [A5] [%] | 6,9 |
| Schmelzintervall [°C] | 975 - 1030 |
| Gießtemperatur [°C] | ca. 1200 |
| WAK [10⁻⁶K⁻¹] 25-500°C | 16,97 |
| WAK [10⁻⁶K⁻¹] 25-600°C | 17,44 |

Es handelt sich somit um eine Edelmetalllegierung des Typs 4 gemäß EN ISO 8891:2000. Der Fachmann wird zur Herstellung einer erfindungsgemäßen Legierung die Legierungsbestandteile vorzugsweise so auswählen, dass den Erfordernissen der EN ISO 8891:2000 entsprochen wird.

Erfindungsgemäße Legierungen erfüllen zudem regelmäßig die Anforderungen der DIN EN ISO 9693:2000, welche Legierungen zur Herstellung keramisch verblendbarer Dentalrestaurationen betrifft (siehe dazu auch die Beispiele unten).

Die erfindungsgemäße Legierung, insbesondere in einer bevorzugten Ausgestaltung, zeichnet sich durch die folgenden Eigenschaften besonders positiv
- hohe Passgenauigkeit der aus der Legierung gegossenen Gerüste (Dentalrestaurationen);
- besonders gute Be-/Verarbeitungseigenschaften;
- hohe Korrosionsresistenz;
- günstige Gießzeitpunkterkennung (Zahntechniker sieht, wann er den Guss auslösen muss);
- schmales Schmelzintervall (vorteilhaft beim Aufschmelzen; geringe Gefahr der Entmischung beim Erstarren)
- Legierung bildet dünnflüssige Schmelze;
- Schmelze der erfindungsgemäßen Legierung besitzt günstiges Ausfließverhalten (daraus resultiert ein hohes Formfüllungsvermögen der Schmelze);
- Legierung ist verblendbar mit allen gängigen Verblendkunststoffen;
- Legierung ist verblendbar mit Verblendkeramiken, die einen WAK im Bereich 15-17 [10⁻⁶K⁻¹] besitzen.

Beim Verblenden mit Keramik besitzt die erfindungsgemäße Legierung die folgenden Vorteile:
- günstiger WAK;
- günstige chemische Haftung (Indium und Mangan wirken hier vorteilhaft);
- hohe Warmfestigkeit (ein Gerüst aus der erfindungsgemäßen Legierung verzieht sich nicht während der Brände);
- alle marktüblichen Keramiken mit einem WAK im Bereich von 15-17 [10⁻⁶ K⁻¹] sind verwendbar;
- preisgünstig

Die Erfindung betrifft auch eine keramisch verblendete Dentalrestauration, umfassend:
- ein Dentalgerüst aus einer erfindungsgemäßen Legierung sowie
- eine auf das Dentalgerüst aufgebrannte Dentalkeramik mit einem WAK im Bereich von 15-17 [10⁻⁶K⁻¹].

Schließlich betrifft die Erfindung auch die Verwendung einer erfindungsgemäßen Legierung zur Herstellung einer keramisch verblendeten Dentalrestauration.

Hinsichtlich der beiden letztgenannten Erfindungsaspekte (Dentalrestauration; Verwendung) gilt hinsichtlich bevorzugter Ausgestaltungen der erfindungsgemäßen Legierungen das Vorgesagte entsprechend.

Nachfolgend wird die Erfindung anhand eines Ausführungsbeispieles näher beschrieben:

### Ausführungsbeispiel:

### 1. Herstellung eines 8-gliedrigen Brückengerüstes:

Es wurde aus Wachs eine 8-gliedrige Oberkiefer-Brücke modelliert; als Modell diente dabei eine reale Patientensituation. Die Mindest-Wandstärke war jeweils 0,4 mm. Es wurden die anatomischen Formen berücksichtigt, so dass der Hauptanteil der Restauration später, nach dem Guss, aus Metall bestand.

Die hergestellte Wachsmodellation wurde in eine phosphatgebundene Einbettmasse eingebettet.

Die resultierende Muffel wurde dann auf eine Temperatur von 900 °C gebracht (Vorwärmtemperatur) und dort für 60 min gehalten.

Der Guss erfolgte in einer induktionsgeheizten Vakuum-Druck-Gussmaschine (Nautilus® MC+/Fa. BEGO, Programm 290) unter Verwendung von Gußplättchen einer erfindungsgemäßen Legierung der folgenden Zusammensetzung:

| | | |
|---|---|---|
| Silber | Ag | 40,5 Gew.-%, |
| Gold | Au | 38,1 Gew.-%, |
| Palladium | Pd | 13,0 Gew.-%, |
| Indium | In | 8,0 Gew.-%, |
| Mangan | Mn | 0,3 Gew.-%, |
| Tantal | Ta | 0,1 Gew.-%, |

Die Gußplättchen wurden in üblicher Weise erhitzt. Der Guß wurde ca. 10 s nach dem Eintauchen der letzten festen Bestandteile der Gußplättchen in die Schmelze manuell ausgelöst. Der Gießzeitpunkt war durch Verwendung von Schmelzpulver sehr günstig zu erkennen, da eine zwischenzeitlich vorhandene Oxidhaut eindeutig aufriss.

Nach dem Abkühlen der Muffel wurde die Einbettmasse grob mechanisch entfernt. Danach wurde das erhaltene Brückengerüst mit Korund der Körnung 110 µm (Korox® 110/Fa. BEGO) bei 2 bar abgestrahlt. Abschließend wurde die Oberfläche des Brückengerüstes mit einer feinverzahnten Hartmetallfräse bearbeitet. Aufgrund der niedrigen Härte und der guten Spanbarkeit (Bearbeitbarkeit) der eingesetzten Legierung gestaltete sich das Ausarbeiten sehr angenehm für den Zahntechniker.
Die Passung des Gerüstes war der von hochgoldhaltigen Legierungen ebenbürtig. Das Fügen (Löten oder Laserschweißen) ist möglich

### 2. Verblenden des Brückengerüstes mit Dentalkeramik mittels Wash- und Grundmassebrand:

Es wurde ein Oxidbrand (10 min bei 780 °C) zur Erzeugung einer homogenen und mit Indium und Mangan als Haftoxid angereicherten Oberfläche durchgeführt.

Vor dem keramischen Verblenden wurde die Oberfläche des Brückengerüstes (aus 1. oben) nochmals, so wie unter 1. beschrieben, abgestrahlt und abgedampft, um die Oberfläche für einen folgenden Washbrand zu konditionieren.

Der Washbrand erfolgte nach Auftrag einer dünnen Suspension einer Verblendkeramik des Typs Response (Fa. Vita). Der Auftrag war dabei nicht deckend.

Es wurde dann nach Auftrag einer deckenden Schicht Pulver-Opaker des Typs Response (Fa. Vita) ein Grundmasse-Brand durchgeführt.

Für die Durchführung des Wash- und des Grundmasse-Brandes wurde, soweit hier nicht anders angegeben, gemäß der Verarbeitungsanleitung des Keramik-Herstellers (Vita) verfahren. Es wurden die Temperaturen und Zeiten verwendet, die in der unten stehenden Tabelle angegeben sind. Als Brennofen diente ein Vakumat 300 (Fa. Vita).

Auf Brände des Typs "Schultermassebrand mit Margin" und "Glanzbrand mit Akzentfluid" (nach dem Grundmassebrand) wurde im Rahmen des Ausführungsbeispieles verzichtet. Solche Brände können aber ergänzend durchgeführt werden.

Gemäß der nachfolgenden Tabelle wurden ergänzend folgende Brände durchgeführt: Oxid-Brand, Wash-Brand (1. Grundmassebrand), Grundmasse-Brand (2. Grundmasse-Brand)1. Dentinbrand, 2. Dentinbrand, Korrekturbrand und Glanzbrand. Dabei wurden wieder Keramikmaterialien des Typs Response (Fa. Vita) eingesetzt.

Die Verbundfestigkeit wurde in In-vitro Versuchen (Abschlag-Test, Abschreck-Test und Biegeversuch gemäß DIN EN ISO 9693:2000) bestimmt. Dabei wurden sämtliche Anforderungen deutlich übertroffen.

Die unverblendeten Anteile (Kronenränder, aber auch unverblendete Kronen) ließen sich sehr einfach polieren. Der schnell erreichte Glanz erfüllt alle Ansprüche an die Ästhetik und bietet dem Anheften von z. B. Speiseresten und der Plaquebildung großen Widerstand entgegen.

**Tabelle mit den Parametern für die keramischen Brände:**

| Brand | Vorwärm-temperatur [°C] | Haltezeit [min] | Heizzeit [min] | Aufheizrate [°C/min] | Endtemperatur [°C] | Haltezeit [min] | Gesamt-vakuumzeit [min] |
|---|---|---|---|---|---|---|---|
| Washbrand | 400 | - | 4 | 55 | 780 | 10 | 16:54 |
| Grundmasse | 400 | 2 | 4 | 55 | 800 | 1 | 8:05 |
| 1. Dentinbrand | 400 | 2 | 6 | 55 | 790 | 1 | 7:05 |
| 2. Dentinbrand | 400 | 6 | 6 | 55 | 770 | 1 | 6:43 |
| Korrekturbrand | 400 | 6 | 6 | 55 | 770 | 1 | 6:43 |
| Glanzbrand | 400 | - | 4 | 55 | 770 | 1 | - |

## Patentansprüche

1. Aufbrennfähige Legierung zur Herstellung keramisch verblendeter Dentalrestaurationen, bestehend aus:
| | |
|---|---|
| Silber | 40-45 Gew.-%, |
| Gold | 37,5-45 Gew.-%, |
| Palladium | 0-14,5 Gew.-%, |
| Indium, Zink, Zinn, Kupfer, Lanthanoide, Scandium, Lanthan | insgesamt 5,5-15 Gew.-%, |
| Mangan | 0,1-5 Gew.-%, |
| Tantal, Platin, Iridium, Osmium, Ruthenium, Rhodium, Rhenium, Titan, Niob, Zirkonium, Wolfram, Vanadium | insgesamt 0,05-5 Gew.-%, |
| Gallium, Eisen | 0-5 Gew.-%, |
| sonstige Metalle, Halbmetalle und Verunreinigungen | 0-1 Gew.-%, |
wobei die Gewichtsprozentangaben jeweils bezogen sind auf das Gesamtgewicht der Legierung.

2. Legierung nach Anspruch 1, umfassend:
| | |
|---|---|
| Silber | 40-44 Gew.-%, vorzugsweise 40,3-43,0 Gew.-% |
| und/oder | |
| Gold | 38-42 Gew.-% |
| und/oder | |
| Palladium | 12,5-14,5 Gew.-%. |

3. Legierung nach einem der vorangehenden Ansprüche, umfassend:
| | |
|---|---|
| Indium, Zink, Zinn, Kupfer, Lanthanoide, Scandium, Lanthan | insgesamt 5,5-10 Gew.-% |

4. Legierung nach einem der Ansprüche 1 bis 3, umfassend;
| | |
|---|---|
| Indium | 5-15 Gew.-%, vorzugsweise 5,5-10 Gew.-%. |

5. Legierung nach einem der vorangehenden Ansprüche, umfassend:
| | |
|---|---|
| Mangan | 0,1-1 Gew,-%. |

6. Legierung nach einem der vorangehenden Ansprüche, umfassend:
| | |
|---|---|
| Tantal, Platin, Iridium, Osmium, Ruthenium, Rhodium, Rhenium, Titan, Niob, Zirkonium, Wolfram, Vanadium | insgesamt 0,05-0,2 Gew.-%. |

7. Legierung nach einem der vorangehenden Ansprüche, umfassend:
| | |
|---|---|
| Tantal | 0,05-5 Gew.-%, vorzugsweise 0,05-0,2 Gew.-%. |

8. Keramisch verblendete Dentalrestauration, umfassend:
- ein Dentalgerüst aus einer Legierung nach einem der Ansprüche 1-7 sowie
- eine auf das Dentalgerüst aufgebrannte Dentalkeramik mit einem WAK im Bereich von 15-17 [10⁻⁶K⁻¹].

9. Verwendung einer Legierung nach einem der Ansprüche 1-7 zur Herstellung einer keramisch verblendeten Dentalrestauration.

## Claims

1. An alloy capable of being fired for the production of dental restorations faced with ceramic material, consisting of:
| | |
|---|---|
| silver | from 40 to 45 % by weight, |
| gold | from 37·5 to 45 % by weight, |
| palladium | from 0 to 14·5 % by weight, |
| indium, zinc, tin, copper, lanthanoids, scandium, lanthanum | altogether from 5·5 to 15 % by weight, |
| manganese | from 0·1 to 5 % by weight, |
| tantalum, platinum, iridium, osmium, ruthenium, rhodium, rhenium, titanium, niobium, zirconium, tungsten, vanadium | altogether from 0·05 to 5 % by weight, |
| gallium, iron | from 0 to 5 % by weight, |
| other metals, semi-metals and impurities | from 0 to 1 % by weight, |
wherein the figures of the percentages by weight relate to the total weight of the alloy in each case.

2. An alloy according to Claim 1, consisting of:
| | |
|---|---|
| silver | from 40 to 44 % by weight, preferably from 40·3 to 43·0 % by weight, |
| and/or | |
| gold | from 38 to 42 % by weight, |
| and/or | |
| palladium | from 12·5 to 14·5 % by weight. |

3. An alloy according to one of the preceding Claims, consisting of:
| | |
|---|---|
| indium, zinc, tin, copper, lanthanoids, scandium, lanthanum | altogether from 5·5 to 10 % by weight. |

4. An alloy according to any one of Claims 1 to 3, consisting of:
| | |
|---|---|
| indium | from 5 to 15 % by weight, preferably from 5·5 to 10 % by weight. |

5. An alloy according to any one of the preceding Claims, consisting of:
| | |
|---|---|
| manganese | from 0·1 to 1 % by weight. |

6. An alloy according to any one of the preceding Claims, consisting of:
| | |
|---|---|
| tantalum, platinum, iridium, osmium, ruthenium, rhodium, rhenium, titanium, niobium, zirconium, tungsten, vanadium altogether | from 0·05 to 0·2 % by weight. |

7. An alloy according to any one of the preceding Claims, consisting of:
| | |
|---|---|
| tantalum | from 0·05 to 5 % by weight, preferably from 0·05 to 0·2 % by weight. |

8. A dental restoration faced with ceramic material, comprising:
- a dental frame of an alloy according to any one of Claims I to 7, and
- a dental ceramic material fired onto the dental frame with a CTE in the range of from 15 to 17 [10⁻⁶ K⁻¹].

9. Use of an alloy according to any one of Claims 1 to 7 for the production of a dental restoration faced with ceramic material.

## Revendications

1. Alliage apte à la cuisson destiné à produire des restaurations dentaires à parement céramique, constitué de :
| | |
|---|---|
| Argent | 40 à 45% en poids, |
| Or | 37,5 à 45% en poids, |
| Palladium | 0 à 14,5% en poids, |
| Indium, zinc, étain, cuivre, lanthanides, scandium, lanthane en tout | 5,5 à 15% en poids, |
| Manganèse | 0,1 à 5% en poids, |
| Tantale, platine, iridium, osmium, ruthénium, rhodium, rhénium, titane, niobium, zirconium, tungstène, vanadium en tout | 0,05 à 5% en poids, |
| Gallium, fer | 0 à 5% en poids, |
| Autres métaux, métalloïdes et impuretés | 0 à 1% en poids, |
les pourcentages indiqués étant rapportés à chaque fois au poids total de l'alliage.

2. Alliage selon la revendication 1, comprenant :
| | |
|---|---|
| Argent | 40 à 44% en poids, |
| | de préférence 40,3 à 43,0% en poids |
| et/ou | |
| Or | 38 à 42% en poids |
| et/ou | |
| Palladium | 12,5 à 14,5% en poids. |

3. Alliage selon l'une des revendications précédentes, comprenant :
| | |
|---|---|
| Indium, zinc, étain, cuivre, lanthanides, scandium, lanthane en tout | 5,5 à 10% en poids |

4. Alliage selon l'une des revendications 1 à 3, comprenant :
| | |
|---|---|
| Indium | 5 à 15% en poids, |
| | de préférence 5,5 à 10% en poids. |

5. Alliage selon l'une des revendications précédentes, comprenant :
| | |
|---|---|
| Manganèse | 0,1 à 1% en poids |

6. Alliage selon l'une des revendications précédentes, comprenant :
| | |
|---|---|
| Tantale, platine, iridium, osmium, ruthénium, rhodium, rhénium, titane, niobium, zirconium, tungstène, vanadium en tout | 0,05 à 0,2% en poids. |

7. Alliage selon l'une des revendications précédentes, comprenant :
| | |
|---|---|
| Tantale | 0,05 à 5% en poids, |
| | de préférence 0,05 à 0,2% en poids. |

8. Restauration dentaire à parement céramique, comprenant :
- une ossature dentaire faite d'un alliage selon l'une des revendications 1 à 7, ainsi que
- une céramique dentaire, déposée par cuisson sur l'ossature dentaire, présentant un coefficient de dilatation thermique dans la plage de 15 à 17 [10⁻⁶K⁻¹].

9. Utilisation d'un alliage selon l'une des revendications 1 à 7 pour produire une restauration dentaire à parement céramique.
